# EUROPEAN PATENT APPLICATION

(11) **EP 4 497 464 A1**
(43) Date of publication of application: **29.01.2025**
(21) Application number: 24189325.4
(22) Date of filing: 18.07.2024
(51) Int. Cl.: A61M 39/06

(54) **HEMOSTATIC SPRINGS AND SEALS**

(30) Priority: 28.07.2023 US 202363529524 P; 10.04.2024 US 202418631385
(71) Applicant: Medtronic, Inc., Minneapolis, MN 55432 (US)
(72) Inventor: DUNLEA, Jake, Galway (IE)
(74) Representative: Zimmermann & Partner Patentanwälte mbB

(57) **Abstract**

Hemostatic springs and hemostatic seals comprising hemostatic springs facilitate introducing a surgical device into the vasculature of a patient during a surgical procedure. Each hemostatic spring comprises a radial array of spring segments positioned on a circular path about a central axis. Each hemostatic seal is configured to reduce an axial length of the hemostatic spring to constrict the radial array of spring segments to reduce a cross-sectional area of the central passage. Methods include axially inserting the surgical device into the hemostatic seal while compression from the hemostatic seal minimizes fluid leakage. Methods can further include reducing a frictional force of inserting the device into the hemostatic seal by reducing the axial compression to dilate the hemostatic spring.

## Description

### CROSS-REFERENCE TO RELATED APPLICATIONS

This application claims the benefit of U.S. Provisional Patent Application Serial No. 63/529,524, filed July 28, 2023, the entire content of which is incorporated herein by reference.

### FIELD

The present disclosure relates generally to hemostatic springs, hemostatic seals, and methods and, more particularly, to hemostatic springs and hemostatic seals with hemostatic springs comprising a radial array of spring segments and methods of introducing a device into a vasculature of a patient by axially inserting the device into a hemostatic seal.

### BACKGROUND

Transcatheter techniques have been developed to provide minimally invasive procedures to introduce medical devices into a patient by passing the medical device through the vasculature of the patient to the target site. Typical introducer systems include an introducer sheath for introduction of a device by way of a transcatheter. The device is known to be passed through a hemostatic valve to prevent blood loss from the introducer sheath when inserting the medical device into the sheath. However, the known biasing mechanisms of conventional hemostatic valves can resist insertion of the device through the valve. The resistance can complicate the procedure and may damage the device or components of the delivery device.

### SUMMARY

The following presents a simplified summary of the disclosure to provide a basic understanding of some aspects described in the detailed description.

Features of the present disclosure provides hemostatic springs and seals that can facilitate dynamic changes of the forces being applied by the hemostatic spring while inserting the device through the hemostatic seal. Providing for dynamic changing of hemostatic spring force can provide the clinician with greater control of the surgical procedure to provide the correct balance of pressure to resist blood loss while minimizing resistance to device insertion through the hemostatic seal to reduce complications and prevent damage to the medical device or components of the delivery device.

In aspects, a hemostatic spring comprises a first end portion, a second end portion opposite the first end portion, and a radial array of spring segments positioned on a circular path about a central axis. Each spring segment of the radial array of spring segments comprises a first end, a second end, and a central location positioned between the first end and the second end. The first end of each spring segment is attached to the first end portion and each spring segment tapers toward the central location in a first direction of the central axis extending from the first end portion toward the second end portion. A second end of each spring segment is attached to the second end portion and each spring segment tapers toward the central location in a second direction of the central axis opposite the first direction. The central location of each spring segment is spaced on the circular path from the central location of each adjacent spring segment of the radial array of spring segments when a force is not applied to the radial array of spring segments.

In further aspects, a hemostatic seal comprises a hemostatic spring comprising a first end portion, a second end portion opposite the first end portion, and a radial array of spring segments positioned on a circular path about a central axis. Each spring segment of the radial array of spring segments comprises a first end, a second end, and a central location positioned between the first end and the second end. The first end of each spring segment is attached to the first end portion and each spring segment tapers toward the central location in a first direction of the central axis extending from the first end portion toward the second end portion. A second end of each spring segment is attached to the second end portion and each spring segment tapers toward the central location in a second direction of the central axis opposite the first direction. The central location of each spring segment is spaced on the circular path from the central location of each adjacent spring segment of the radial array of spring segments when a force is not applied to the radial array of spring segments. The hemostatic seal further comprises a proximal mount comprising a proximal aperture aligned with the central axis extending through a central passage of the hemostatic spring, wherein the first end portion of the hemostatic spring is mounted to the proximal mount. The hemostatic seal further comprises a distal mount comprising a distal aperture aligned with the central axis, wherein the second end portion of the hemostatic spring is mounted to the distal mount. The hemostatic seal is configured to reduce the distance between the proximal mount and the distal mount along the central axis to constrict the radial array of spring elements to reduce a cross-sectional area of the central passage.

In still further aspects, a method of introducing a device into a vasculature of a patient comprising percutaneously inserting an introducer sheath into the vasculature of the patient wherein the introducer sheath is provided with a hemostatic seal comprising a hemostatic spring under an axial compression to minimize leakage of fluid from the introducer sheath. The method further comprises axially inserting the device into the hemostatic seal, and reducing a frictional force of inserting the device into the hemostatic seal by reducing the axial compression to dilate the hemostatic spring. The method further comprises passing the device into the introducer sheath from the hemostatic seal and passing the device from the introducer sheath to the vasculature of the patient.

### BRIEF DESCRIPTION OF THE DRAWINGS

These and other features, aspects and advantages are better understood when the following detailed description is read with reference to the accompanying drawings, in which:
**FIG. 1** is a schematic view of a hemostatic seal in accordance with aspects of the disclosure being used during a surgical procedure;
**FIG. 2** is a schematic cross-sectional view of the hemostatic seal of **FIG. 1****;**
**FIG. 3** is a schematic cross-sectional view of the hemostatic seal of **FIG. 2** with a surgical device being inserted through the hemostatic seal;
**FIG. 4** is a side view of a hemostatic spring in accordance with embodiments of the disclosure when an axial compressive force is not applied compress the hemostatic spring;
**FIG. 5** is an end view of the hemostatic spring along view 5-5 of **FIG. 4****;**
**FIG. 6** is a side view of the hemostatic spring of **FIG. 4** when an axial compressive force is applied to compress the hemostatic spring;
**FIG. 7** is an end view of the hemostatic spring along view 7-7 of **FIG. 6****;**
**FIG. 8** is a side view of a hemostatic spring in accordance with further embodiments of the disclosure when an axial compressive force is applied to compress the hemostatic spring;
**FIG. 9** is a side view of the hemostatic spring of **FIG. 8** when an axial compressive force is not applied to compress the hemostatic spring;
**FIG. 10** is a side schematic view representing hemostatic springs of further embodiments of the disclosure when an axial compressive force is applied to compress the hemostatic springs;
**FIG. 11** is a side view of one embodiment of the hemostatic spring of **FIG. 10** when an axial compressive force is not applied to compress the hemostatic spring;
**FIG. 12** is a side view of another embodiment of the hemostatic spring of **FIG. 10** when an axial compressive force is not applied to compress the hemostatic spring;
**FIG. 13** is a schematic cross-sectional view of a hemostatic seal in accordance with embodiments of the disclosure;
**FIGS. 14-17** illustrate introducing a surgical device through the hemostatic seal of **FIG. 13** during a surgical procedure;
**FIG. 18** is a schematic cross-sectional view of a hemostatic seal in accordance with embodiments of the disclosure;
**FIG. 19** illustrates introducing a surgical device through the hemostatic seal of **FIG. 18** during a surgical procedure;
**FIG. 20** is a schematic cross-sectional view of a hemostatic seal in accordance with embodiments of the disclosure; and
**FIG. 21** illustrates introducing a surgical device through the hemostatic seal of **FIG. 20** during a surgical procedure.

### DETAILED DESCRIPTION

Aspects will now be described more fully hereinafter with reference to the accompanying drawings in which example aspects are shown. Whenever possible, the same reference numerals are used throughout the drawings to refer to the same or like parts. However, this disclosure may be embodied in many different forms and should not be construed as limited to the aspects set forth herein.

The terms "distal" and "proximal" are used in the following description with respect to a position or direction relative to an expected position of a treating clinician. "Distal" or "distally" are a position distant from or in a direction away from the clinician. "Proximal" and "proximally" are a position near or in a direction toward the clinician. In addition, as used herein, the terms "outward" or "outwardly" refer to a position radially away from a central axis of the hemostatic spring and the terms "inward" or "inwardly" refer to a position radially toward a central axis of the hemostatic spring.

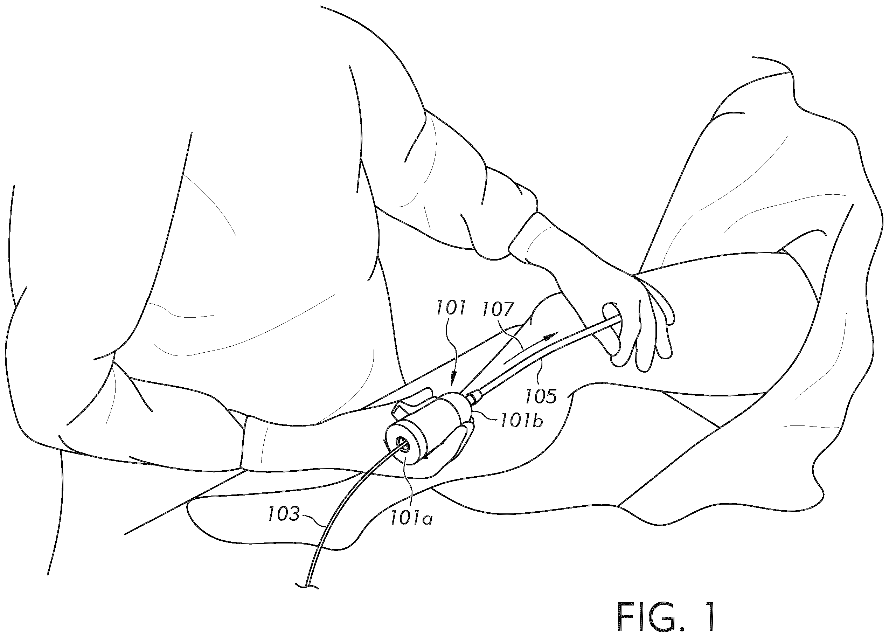

**FIG. 1** is a schematic view of a hemostatic seal **101** in accordance with aspects of the disclosure being used during a surgical procedure to insert a surgical device into the vasculature of a patient with a catheter **103.** As shown, a catheter is inserted in a distal direction **107** into a proximal end **101a** of the hemostatic seal **101** while an introducer sheath **105** extends in the distal direction **107** from a distal end **101b** of the hemostatic seal **101.** The distal end of the introducer sheath **105** can be inserted into the vasculature of a patient where the surgical device is to be introduced.

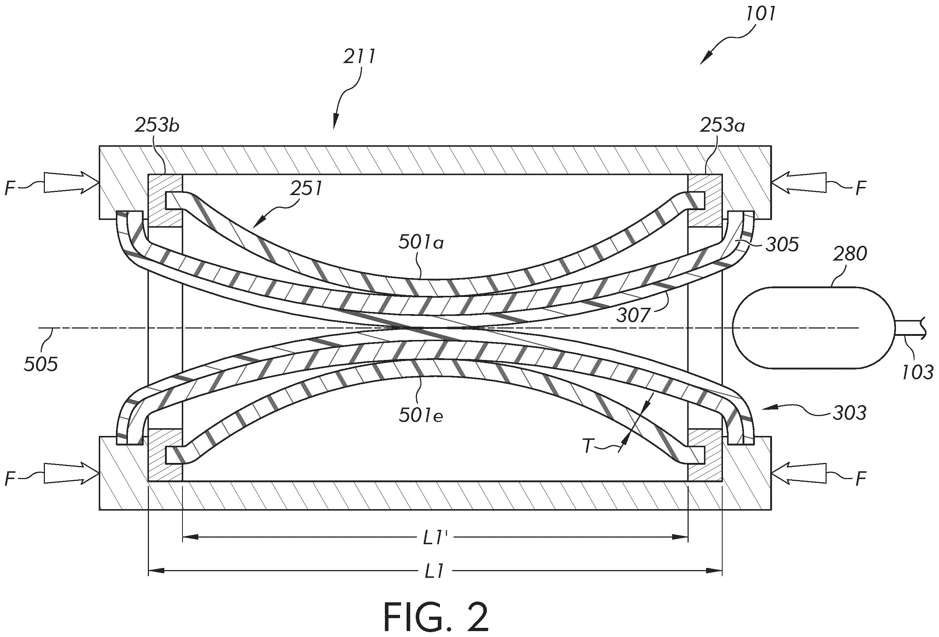

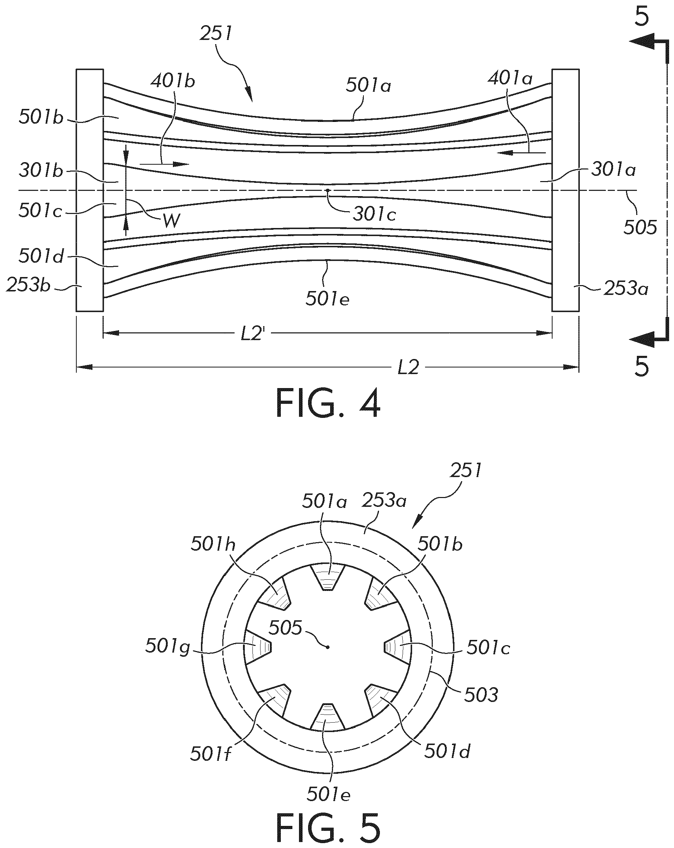

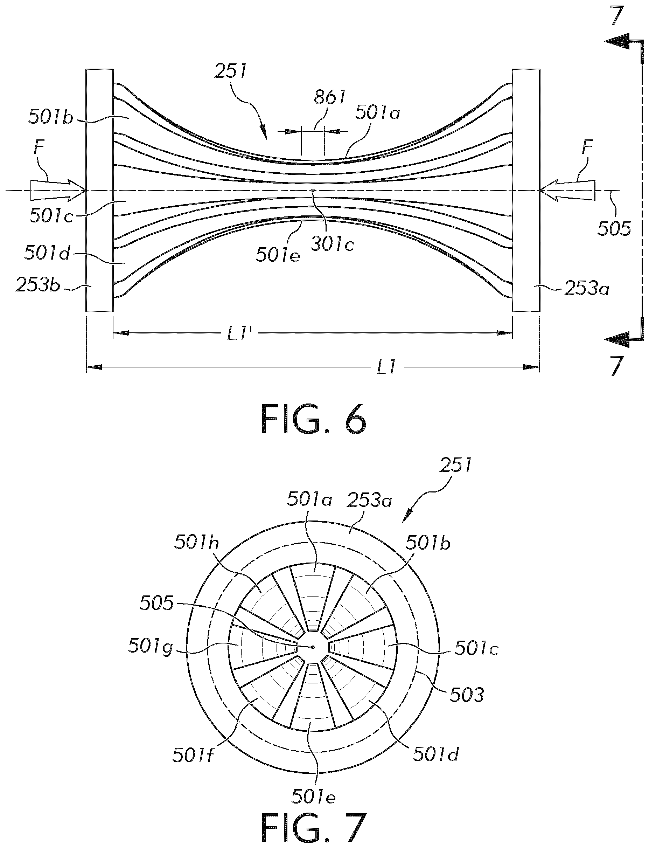

**FIG. 2** illustrates a schematic cross-sectional view of an embodiment of the hemostatic seal **101** of **FIG. 1** comprising a housing **211** containing a hemostatic spring **251.** The hemostatic spring **251** comprises a first end portion **253a** and a second end portion **253b** opposite the first end portion **253a.** As shown in **FIGS. 5** **and** **7****,** the hemostatic spring **251** comprises a radial array of spring segments **501a-h** positioned on a circular path **503** about a central axis **505** of the hemostatic spring **251.** As shown, the spring segments **501a-h** can be equally spaced from one another about the circular path **503** to equalize the resultant force applied by the spring segments in axial compression. Eight spring segments are illustrated although any plurality of spring segments may be provided in further embodiments.

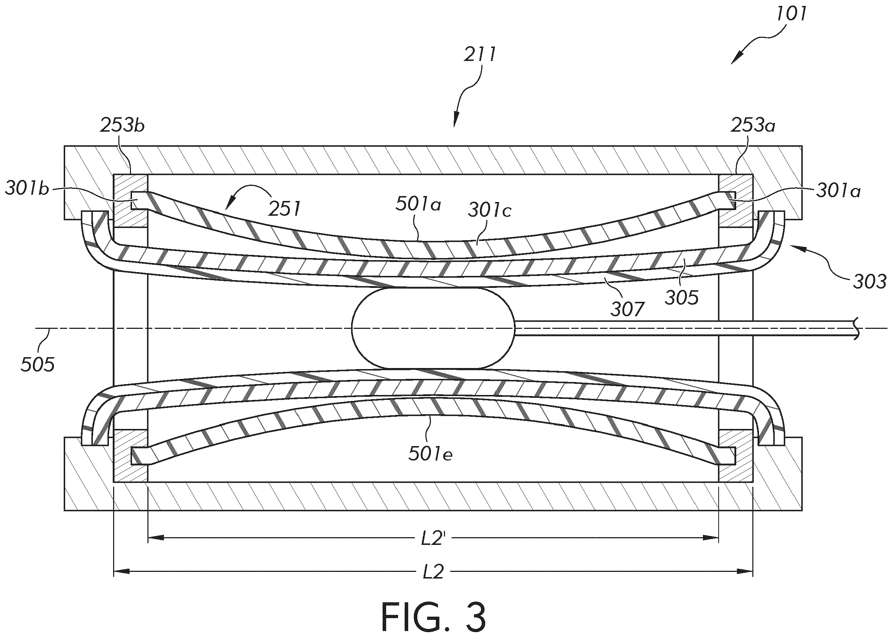

In some embodiments, as shown, the spring segments **501a-h** can be substantially identical to one another. As such, the discussion of any spring segment can equally apply to all spring segments. As shown in **FIG. 3****,** each spring segment **501a-h** of the radial array of spring segments **501a-h** comprises a first end **301a,** a second end **301b,** and a central location **301c** positioned between the first end **301a** and the second end **301b.** In some embodiments, as shown in **FIG. 3****,** the first end **301a** of each spring segment **501a-h** is attached to the first end portion **253a** and a second end **301b** of each spring segment 501**a-h** is attached to the second end portion **253b** of the hemostatic spring **251.**

Each spring segment **501a-h** of the radial array of spring segments **501a-h** may taper in a direction from each end **301a, 301b** toward the central location **301c** of the spring segment. For example, with reference to spring segment **501c** in **FIG. 4****,** each spring segment **501a-h** tapers toward the central location **301c** in a first direction **401** a of the central axis **505** extending from the first end portion **253a** toward the second end portion **253b,** and tapers toward the central location **301c** in a second direction **401b** of the central axis **505** opposite the first direction **401a.** In some embodiments, the tapering of the spring segments **501a-h** can be continuously tapering or discontinuously tapering (e.g., periodically, or intermittently tapering). In the illustrated embodiment of **FIGS. 4-7****,** each spring segment spring segment **501a-h** continuously tapers toward the central location **301c** in the first direction **401a** and the second direction **401b.** Still further, in some embodiments, the tapering may begin at a location spaced from the corresponding end portion or may begin at the end portion. In the illustrated embodiment of **FIGS. 4-7****,** the tapering begins at the end portion and continuously tapers until it reaches the central location **301c** (e.g., the midpoint of the spring segment in the embodiment of **FIGS. 4-7**).

Throughout the disclosure, as shown in **FIG. 2****,** the length "**L1**" is the overall length of the fully compressed hemostatic spring **251** while the length **L1'** is the length of a spring segment of the fully compressed hemostatic spring **251** that is considered the distance extending in the direction of the central axis **505** between the first end portion **253a** and the second end portion **253b.** As shown in **FIG. 3****,** the length **"L2"** is the overall length of an uncompressed hemostatic spring **251** while the length **L2'** is the length of a spring segment of an uncompressed hemostatic spring **251** that is considered the distance extending in the direction of the central axis **505** between the first end portion **253a** and the second end portion **253b.** The thickness **"T"** of the spring segment is considered the cross-sectional thickness of the spring segment along a cross-sectional plane including the central axis **505** of the hemostatic spring **251** and the central axis of the spring segment. The width **"W"** of the spring segment is considered the distance across the spring segment in a width direction that is perpendicular to the direction of the thickness **"T"** and perpendicular to a direction of the central axis **505.**

Unless otherwise stated, for purposes of this disclosure, tapering of the spring segments throughout the disclosure means tapering of the width **"W"** of the spring segment and/or tapering of the thickness **"T"** of the spring segment. For example, as shown, in **FIG. 4****,** the tapering of the spring segments **501a-h** can comprise tapering of the width **"W"** while as shown in **FIG. 2****,** the thickness **"T"** may not be tapered although the thickness may be tapered (in addition or alternative to the width) in further embodiments.

As shown in **FIG. 3****,** the first end **301a** and the second end **301b** of the spring segments **501a-h** may each be received in a corresponding mounting socket of the first and second end portions **253a, 253b.** In some embodiments the first and second ends **301a, 301b** can be permanently fixedly attached within the mounting sockets, e.g., by interference fit, welding, or other fastening technique. In further embodiments, the spring segments and end portions may be formed as a single monolithic member, for example, by injection molding.

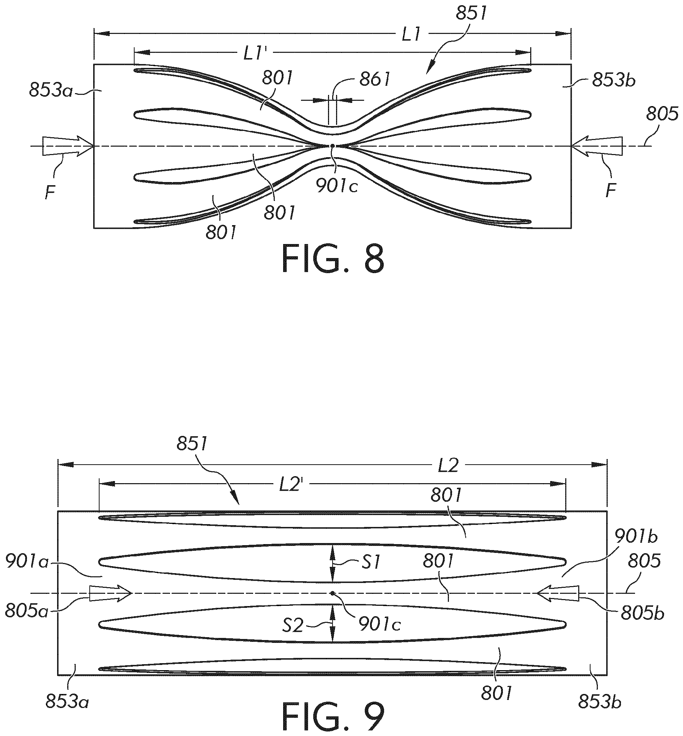

The hemostatic spring can be formed from a wide range of flexible, resilient materials such as polymeric material, spring steel, stainless steel. Furthermore, as shown in the embodiment of **FIGS. 2-7****,** the hemostatic spring **251** can comprise multiple components that are assembled together. However, as mentioned previously in alternative embodiments, the hemostatic spring can comprise a single monolithic member. For example, **FIGS. 8-9** disclose another embodiment of a hemostatic spring **851** fabricated from a single one-piece tube. As shown in **FIG. 8****,** the hemostatic spring **851** is configured to be axially compressed to constrict the radial array of spring segments **801** to a minimum cross-sectional area along a midpoint length **861** centered at the midpoint comprising a central location **901c** of the spring segments. Likewise, as shown in **FIG. 6****,** the hemostatic spring **251** is configured to be axially compressed to constrict the radial array of spring segments **501a-h** to a minimum cross-sectional area along a midpoint length **861** centered at the midpoint comprising the central location **301c** of the spring segments.

For purposes of this application, the midpoint length can be experimentally determined by placing the hemostatic spring between two parallel steel plates wherein the central axis of the hemostatic spring is perpendicular to inner contact surfaces of the parallel steel plates. The steel plates are moved together in 0.5 millimeter (mm) increments to incrementally constrict the radial array of spring elements to reduce a cross-sectional area of the central passage. Lengths of the central portion having the minimum cross-sectional area of the central passage is measured after each incremental adjustment of the steel plates. The midpoint length **861** of the hemostatic spring is the length of the minimum cross-sectional area of the central passage at the increment prior to the incremental adjustment that causes failure of the hemostatic spring by plastic deformation or breaking of the hemostatic spring. In some embodiments, the midpoint length **861** of the hemostatic spring can be within a range of from about 1% to about 5% of the length **L1'** of each identical spring segment in the radial array of spring segments.

As shown in **FIGS 8-9****,** the hemostatic spring **851** comprises many features that are similar to the features of the hemostatic spring **251** of **FIGS. 4-7****.** The hemostatic spring **851** comprises a first end portion **853a** and a second end portion **853b** opposite the first end portion **853a.** The hemostatic spring **851** comprises a radial array of spring segments **801** positioned on a circular path about a central axis **805** of the hemostatic spring **851.** As shown, the spring segments **801** can be equally spaced from one another about the circular path to equalize the resultant force applied by the spring segments in axial compression. As can be appreciated, eight spring segments are provided although any plurality of spring segments may be provided in further embodiments.

In some embodiments, as shown, the spring segments **801** can be substantially identical to one another. As such, the discussion of any spring segment will equally apply to all spring segments. As shown in **FIG. 9****,** each spring segment **801** of the radial array of spring segments **801** comprises a first end **901a**, a second end **901b,** and a central location **901c** positioned between the first end **901a** and the second end **901b.** In some embodiments, as shown in **FIG. 9****,** the first end **901a** of each spring segment **801** is attached to the first end portion **853a** and a second end **901b** of each spring segment **801** is attached to the second end portion **853b** of the hemostatic spring **851.**

Each spring segment **801** of the radial array of spring segments **801** may taper in a direction from each end portion **853a, 853b** toward the central location **901c** of the spring segment. For example, with reference to **FIG. 9****,** each spring segment **801** tapers toward the central location **901c** in a first direction **805a** of the central axis **805** extending from the first end portion **853a** toward the second end portion **853b,** and tapers toward the central location **901c** in a second direction **805b** of the central axis **805** opposite the first direction **805a.** In some embodiments, the tapering of the spring segments **801** can be continuously tapering or discontinuously tapering (e.g., periodically, or intermittently tapering). In the illustrated embodiment of **FIGS. 8-9****,** each spring segment spring segment **801** continuously tapers toward the central location **901c** in a first direction **805a** of the central axis **805** extending from the first end portion toward **853a** the second end portion **853b,** and continuously tapers toward the central location **901c** in a second direction **805b** of the central axis **805** opposite the first direction **805a.** Still further, in some embodiments, the tapering may begin at a location spaced from the corresponding end portion or may begin at the end portion. In the illustrated embodiment of **FIGS. 8-9****,** the tapering begins at the end portion and continuously tapers until it reaches the central location **901c** (e.g., the midpoint of the spring segment in the embodiment of **FIGS. 8-9**).

As shown in **FIGS. 8-9****,** in some embodiments, the hemostatic spring **851** is fabricated from a single one-piece tube. As shown in **FIG. 8****,** the hemostatic spring **851** is configured to be axially compressed to constrict the radial array of spring segments **801** to a minimum cross-sectional area along the midpoint length **861** centered at the midpoint comprising the central location **901c** of the spring segments. Likewise, as shown in **FIG. 6****,** the hemostatic spring **251** is configured to be axially compressed to constrict the radial array of spring segments **501a-h** to a minimum cross-sectional area along a midpoint length **861** centered at the midpoint comprising the central location **301c** of the spring segments.

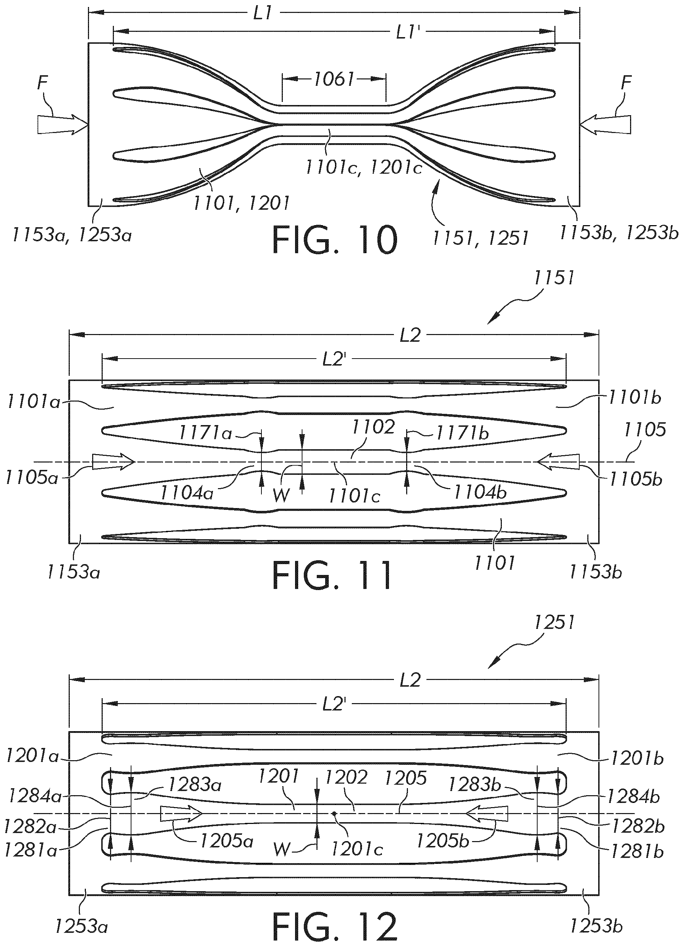

**FIGS. 11-12** disclose alternative embodiments of hemostatic springs **1151, 1251** that can each be fabricated from a single one-piece tube. **FIG. 10** is a schematic representation of the hemostatic springs **1151, 1251** that are axially compressed to constrict the radially array of spring segments **1101, 1201** to a minimum cross-sectional area along a central segment length **1061** of the central segment comprising the central location **1101c, 1201c** of the spring segments **1101, 1201.**

For purposes of this application, the central segment length **1061** can be experimentally determined by placing the hemostatic spring between two parallel steel plates wherein the central axis of the hemostatic spring is perpendicular to inner contact surfaces of the parallel steel plates. The steel plates are moved together in 0.5 millimeter (mm) increments to incrementally constrict the radial array of spring elements to reduce a cross-sectional area of the central passage. Lengths of the central portion having the minimum cross-sectional area of the central passage is measured after each incremental adjustment of the steel plates. The central segment length **1061** of the hemostatic spring is the length of the minimum cross-sectional area of the central passage at the increment prior to the incremental adjustment that causes failure of the hemostatic spring by plastic deformation or breaking of the hemostatic spring. In some embodiments, the central segment length **1061** of the hemostatic spring can be within a range of from greater than 5% to less than 50% of the length **L1'** of each identical spring segment in the radial array of spring segments.

As shown in **FIG. 11****,** the hemostatic spring **1151** a first end portion **1153a** and a second end portion **1153b** opposite the first end portion **1153a.** The hemostatic spring **1151** comprises a radial array of spring segments **1101** positioned on a circular path about a central axis **1105** of the hemostatic spring **1151.** As shown, the spring segments **1101** can be equally spaced from one another about the circular path to equalize the resultant force applied by the spring segments in axial compression. As can be appreciated, eight spring segments are provided although any plurality of spring segments may be provided in further embodiments.

In some embodiments, as shown, the spring segments **1101** can be substantially identical to one another. As such, the discussion of any spring segment will equally apply to all spring segments. As shown in **FIG. 11****,** each spring segment **1101** of the radial array of spring segments **1101** comprises a first end **1101a,** a second end **1101b,** and a central location **1101c** positioned between the first end **1101a** and the second end **1101b.** In some embodiments, the first end **1101a** of each spring segment **1101** is attached to the first end portion **1153a** and a second end **1101**b of each spring segment **1101** is attached to the second end portion **1153b** of the hemostatic spring **1151.**

Each spring segment **1101** of the radial array of spring segments **1101** may taper in a direction from each end portion **1153a, 1153b** toward the central location **1101c** of the spring segment. For example, as shown, each spring segment **1101** tapers toward the central location **1101c** in a first direction **1105a** of the central axis **1105** extending from the first end portion **1153a** toward the second end portion **1153b,** and tapers toward the central location **1101c** in a second direction **1105b** of the central axis **1105** opposite the first direction **1105a.** In some embodiments, the tapering of the spring segments **1101** can be continuously tapering or discontinuously tapering (e.g., periodically, or intermittently tapering). In the illustrated embodiment, each spring segment spring segment **1101** continuously tapers toward the central location **1101c** in the first direction **1105a** of the central axis **1105,** and continuously tapers toward the central location **1101c** in a second direction **1105b** of the central axis **1105.** Still further, in some embodiments, the tapering may begin at a location spaced from the corresponding end portion or may begin at the end portion. In the illustrated embodiment of **FIG. 11****,** the tapering begins at the end portion and continuously tapers until it reaches the central location **1101c.**

As further illustrated in **FIG. 11****,** the central location **1101c** of each spring segment **1101** can comprise a central segment **1102.** In some embodiments, the central segment **1102** can comprise a width **"W"** that is substantially constant along the central axis **1105.** The substantially constant width **"W"** can provide a central portion with a consistent cross-sectional passage dimension along the central segment length **1061** to provide consistent sealing against the surgical device passing through the hemostatic seal. Each spring segment **1101** continuously tapers from the first end **1101a** to a first central waist **1104a** of the central location **1101**c, and continuously tapers from the second end **1101b** to a second central waist **1104b** of the central location **1101c.** As shown, the central location **1101c** therefore comprises the central segment **1102,** the first central waist **1104a,** and the second central waist **1104b,** wherein the central segment **1102** is positioned between the first central waist **1104a** and the second central waist **1104b.** Each spring segment **1101** can continuously taper from the first end **1101a** to the first central waist **1104a** of the central location **1101c,** and continuously taper from the second end **1101b** to the second central waist **1104b** of the central location **1101c,** wherein the central segment **1102** is positioned between the first central waist **1104a** and the second central waist **1104b.** As further illustrated, a width **"W"** of the central segment can be greater than a width **1171a** of the first central waist **1104a** and a width **1171b** of the second central waist **1104b.** Providing the reduced widths at the first and second central waists **1104a, 1104b** can encourage pivoting of the spring segments **1101** when constricting the spring segments **1101** at these locations to achieve the configuration shown in **FIG. 10** while reducing stress concentrations at the pivot points.

**FIG. 12** illustrates another embodiment of the hemostatic spring **1251** schematically illustrated in **FIG. 10****.** As shown, the hemostatic spring **1251** comprises a first end portion **1253a** and a second end portion **1253b** opposite the first end portion **1253a.** The hemostatic spring **1251** comprises a radial array of spring segments **1201** positioned on a circular path about a central axis **1205** of the hemostatic spring **1251.** As shown, the spring segments **1201** can be equally spaced from one another about the circular path to equalize the resultant force applied by the spring segments in axial compression. As can be appreciated, eight spring segments are provided although any plurality of spring segments may be provided in further embodiments.

In some embodiments, as shown, the spring segments **1201** can be substantially identical to one another. As such, the discussion of any spring segment will equally apply to all spring segments. As shown in **FIG. 12****,** each spring segment **1201** of the radial array of spring segments **1201** comprises a first end **1201a,** a second end **1201b,** and a central location **1201c** positioned between the first end **1201a** and the second end **1201b.** In some embodiments, the first end **1201a** of each spring segment **1201** is attached to the first end portion **1253a** and a second end **1201b** of each spring segment **1201** is attached to the second end portion **1253b** of the hemostatic spring **1251.**

Each spring segment **1201** of the radial array of spring segments **1201** may taper in a direction from each end portion **1253a, 1253b** toward the central location **1201c** of the spring segment. For example, as shown, each spring segment **1201** tapers toward the central location **1201c** in a first direction **1205a** of the central axis **1205** extending from the first end portion **1253a** toward the second end portion **1253b,** and tapers toward the central location **1201c** in a second direction **1205b** of the central axis **1205** opposite the first direction **1205a.** In some embodiments, the tapering of the spring segments **1201** can be continuously tapering or discontinuously tapering (e.g., periodically, or intermittently tapering). In the illustrated embodiment, each spring segment spring segment **1201** continuously tapers toward the central location **1201c** in the first direction **1205a** of the central axis **1205,** and continuously tapers toward the central location **1201c** in a second direction **1205b** of the central axis **1205.** Still further, as shown, the tapering can begin at a location spaced from the corresponding end portion or may begin at the end portion. For example, as shown in **FIG. 12****,** the first end **1201a** of each spring segment **1201** is attached at a first end waist **1281a** to the first end portion **1253a** and the second end **1201b** of each spring segment **1201** is attached at a second end waist **1281b** to the second end portion **1253b.** Each spring segment **1201** tapers (e.g., continuously tapers) in the first direction **1205a** from a first intermediate portion **1283a** toward the central location **1201c** wherein the central location **1201c** can comprise a central segment in some embodiments. Each spring segment **1201** further tapers (e.g., continuously tapers) in the second direction **1205b** of the central axis **1205** from a second intermediate portion **1283b** toward the central location **1201c** wherein the central location **1201c** can comprise a central segment in some embodiments.

As further illustrated in **FIG. 12****,** the first end waist **1281a** is positioned between the first end portion **1253a** and the first intermediate portion **1283a.** The second end waist **1281b** is positioned between the second end portion **1253b** and the second intermediate portion **1283b.** As shown, the first intermediate portion **1283a** comprises a maximum width **1284a** that is greater than a minimum width **1282a** of the first end waist **1281a,** and the second intermediate portion **1283b** comprises a maximum width **1284b** greater than a minimum width **1282b** of the second end waist **1281b.** In some embodiments, as shown, the minimum width **1282a** of the first end waist **1281a** and the minimum width **1282b** of the second end waist **1281b** can each be greater than a minimum width **"W"** of the central location **1201c** (e.g., central segment). Furthermore, as shown, in some embodiments, the central location **1201c** of each spring segment **1201** can comprise a central segment **1202** of the corresponding spring segment with a substantially constant width **"W"** along the central axis **1205.** Providing the end waists with reduced minimum width relative to the maximum width of the associated intermediate portion helps reduce stress and plastic deformation of the hemostatic spring when the hemostatic spring is axially compressed to the configuration schematically illustrated in **FIG. 10****.**

As shown in **FIGS. 4-5****, 9****, and** **11-12****,** each embodiment of the hemostatic spring **251, 851, 1151, 1251** provides that the central location **301c, 901c, 1101c, 1201c** of each spring segment **501a-h, 801, 1101, 1201** is spaced on the circular path **503** from the central location **301c, 901c, 1101c, 1201c** of each adjacent spring segment of the radial array of spring segments **501a-h, 801, 1101, 1201** when a force is not applied to the radial array of spring segments **501a-h, 801, 1101, 1201.** For example, as shown in **FIG. 9****,** when the axial forces are not present that compress the hemostatic spring **851,** a space **"S1"** exists on the circular path between the spring segment **801** (i.e., the central spring segment as it appears in **FIG. 9**) and the adjacent spring segment **801** positioned above the central spring segment as it appears in **FIG. 9****.** Further, when axial forces are not present that compress the hemostatic spring **851,** as further shown in **FIG. 9****,** a space **"S2"** exists on the circular path between the central spring segment **801** and the spring segment **801** positioned below the central spring segment as it appears in **FIG. 9****.** Therefore, in a relaxed state, the central locations of the spring segments are spaced apart from one another. In contrast, under axial compression (see opposed forces **"F"** in FIGS. 2, 6, and 8), the central locations constrict to radially contract toward the central axis of the hemostatic spring wherein the spring segments act together to narrow the passage passing through the hemostatic spring. While the spring segments are radially constricted, they also act to radially bias the hemostatic spring back to the original relaxed state. As such, once the compressive axial force **"F"** is removed, each of the central locations radially retract to dilate the passage and thereby return the hemostatic spring back to its original relaxed state.

As mentioned previously, in some embodiments, the hemostatic springs (e.g., hemostatic springs **851, 1151, 1251)** can be fabricated as a one piece spring. For example, in some embodiments, the hemostatic spring can be fabricated from a tube that can then be laser cut or otherwise machined into the desired configuration (e.g., as shown in **FIGS. 8-12**). In such example, the first end portion **853a, 1153a, 1253a,** and the second end portion **853b, 1153b, 1253b** can each comprise a continuous circular cylindrical tube. A thickness of the first end portion, a thickness of the second end portion, and a thickness of each spring segment can also be approximately equal. Furthermore, each spring segment of the radial array of spring segments can be substantially straight and extend parallel to the central axis when a force is not applied to the radial array of spring segments. Although not shown, in some embodiments, a slight crimp may be provided at the center of the spring segments to help initiate inward rather than outward buckling of the spring segments when applying the axial force to the hemostatic spring.

Turning back to **FIG. 3****,** in some embodiments, the hemostatic springs of the present disclosure may comprise one or more internal sleeves **303** that can line an internal passage of the hemostatic spring to help contain fluid and reduce friction between the surgical device and the hemostatic spring. In some embodiments, the internal sleeve **303** of hemostatic spring can comprise an elastomeric sublayer **305** (e.g., silicone) that may provide compliance to seal around the surgical device. In some embodiments, the internal sleeve **303** can further comprise a low friction polymer **307** (e.g., PTFE) to reduce friction and thereby ease passage of the device and reduce the likelihood of snagging on the sleeve.

Embodiments of hemostatic seals are now described that can incorporate any one of the hemostatic springs described throughout this application including the hemostatic springs **251, 851, 1151, 1251.** For purposes of description of the embodiments of the hemostatic seals, the description of the various embodiments of the hemostatic seals will be discussed incorporating the hemostatic spring **851** illustrated in **FIGS. 8-9** with the understanding that the description would similarly or identically apply to all hemostatic seals incorporating any of the other hemostatic springs (e.g., hemostatic springs **251, 1151, 1251).**

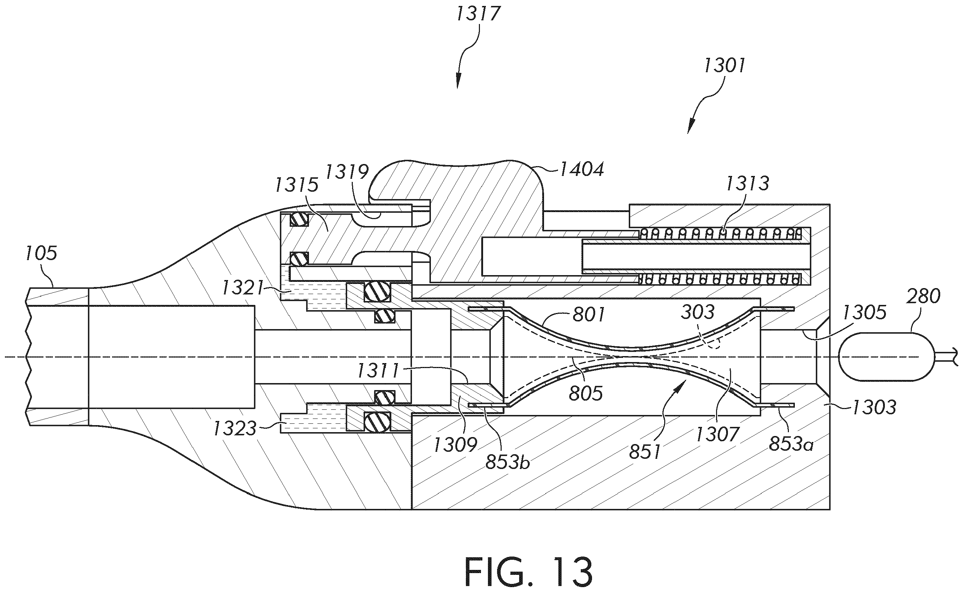

**FIG. 13** illustrates one embodiment of a hemostatic seal **1301** comprising the hemostatic spring **851** described above although any hemostatic spring may be provided in accordance with embodiments of the disclosure. The hemostatic seal **1301** comprises a proximal mount **1303** comprising a proximal aperture **1305** aligned with the central axis **805** extending through a central passage **1307** of the hemostatic spring **851,** wherein the first end portion **853a** of the hemostatic spring **851** is mounted to the proximal mount **1303.** The hemostatic seal **1301** can further comprise a distal mount **1309** comprising a distal aperture **1311** aligned with the central axis **805,** wherein the second end portion **853b** of the hemostatic spring **851** is mounted to the distal mount **1309**

The hemostatic seal **1301** is configured to reduce the distance between the proximal mount **1303** and the distal mount **1309** along the central axis **805** to constrict the radial array of spring elements **801** to reduce a cross-sectional area of the central passage **1307.** As shown in **FIG. 13****,** in one embodiment, a compression spring **1313** is configured to bias at least one of the proximal mount **1303** and the distal mount **1309** to reduce the distance between the proximal mount **1303** and the distal mount **1309** along the central axis **805** to constrict the radial array of spring elements **801.** For example, as shown, the compression spring **1313** biases a linear piston **1315** of an actuator **1317** to the left within the hydraulic cylinder **1319** in the orientation shown in **FIG. 13** to expel the hydraulic fluid **1321** from the hydraulic cylinder **1319** to enter a compression chamber **1323.** The hydraulic fluid **1321** is therefore hydraulically compressed to apply force to the distal mount **1309** and therefore bias the distal mount **1309** reduce the distance between the proximal mount **1303** and the distal mount **1309** along the central axis **805** to constrict the radial array of spring elements **801.**

The actuator **1317** of the hemostatic seal **1301** is also configured to act against the bias of the compression spring **1313** to selectively dilate the radial array of spring segments **801** to increase a cross-sectional area of the central passage **1307.** In some embodiments, the actuator can comprise the illustrated trigger **1404.**

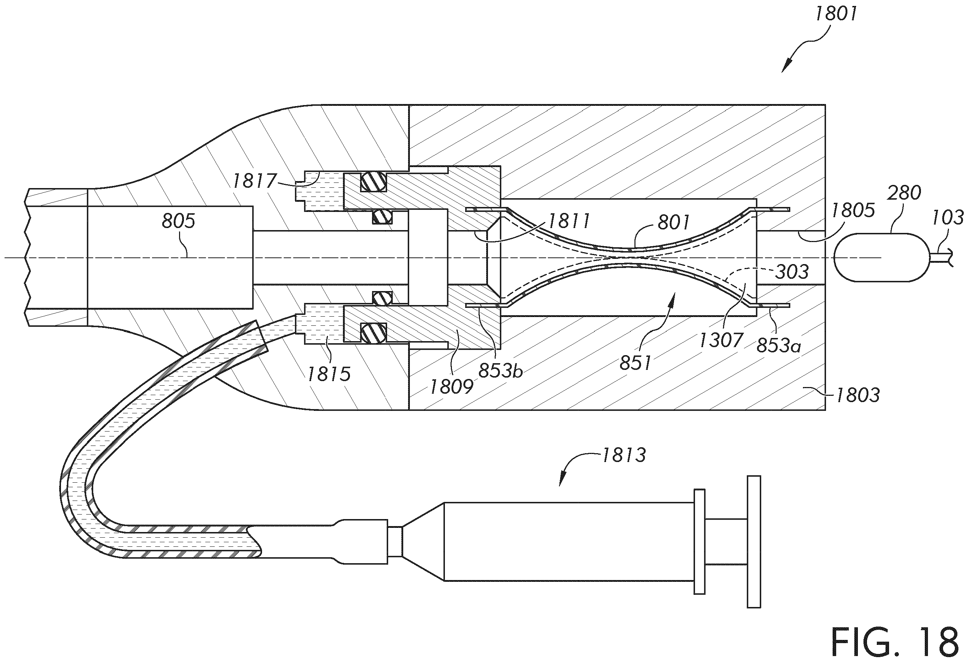

**FIG. 18** illustrates another embodiment of a hemostatic seal **1801** comprising the hemostatic spring **851** described above although any hemostatic spring may be provided in accordance with embodiments of the disclosure. The hemostatic seal **1801** comprises a proximal mount **1803** comprising a proximal aperture **1805** aligned with the central axis **805** extending through a central passage **1307** of the hemostatic spring **851,** wherein the first end portion **853a** of the hemostatic spring **851** is mounted to the proximal mount **1803.** The hemostatic seal **1801** can further comprise a distal mount **1809** comprising a distal aperture **1811** aligned with the central axis **805,** wherein the second end portion **853b** of the hemostatic spring **851** is mounted to the distal mount **1809.**

The hemostatic seal **1801** is configured to reduce the distance between the proximal mount **1803** and the distal mount **1809** along the central axis **805** to constrict the radial array of spring elements **801** to reduce a cross-sectional area of the central passage **1307** to minimize leakage of fluid from the introducer sheath. As shown in **FIG. 18****,** in one embodiment, a syringe **1813** is used to control hydraulic fluid **1815** within a pressure chamber **1817.** As shown, the syringe **1813** can be positioned to pressurize the hydraulic fluid **1815** in the pressure chamber **1817** to bias the distal mount **1809** to reduce the distance between the proximal mount **1803** and the distal mount **1809** along the central axis **805** to constrict the radial array of spring segments **801.** Although not shown, a lock may be provided on the syringe to releasably lock the syringe at a desired adjusted position wherein the clinician can later release it.

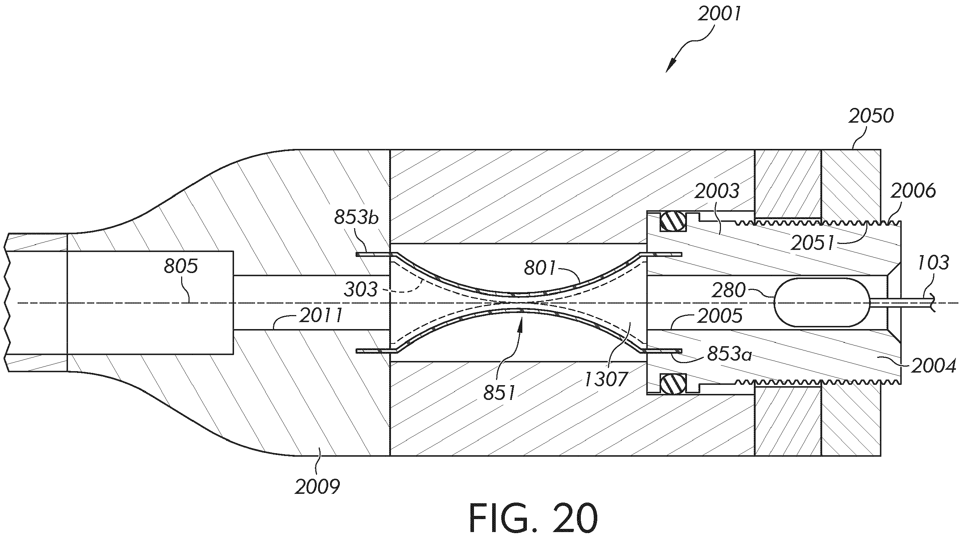

**FIG. 20** illustrates another embodiment of a hemostatic seal **2001** comprising the hemostatic spring **851** described above although any hemostatic spring may be provided in accordance with embodiments of the disclosure. The hemostatic seal **2001** comprises a proximal mount **2003** comprising a proximal aperture **2005** aligned with the central axis **805** extending through a central passage **1307** of the hemostatic spring **851,** wherein the first end portion **853a** of the hemostatic spring **851** is mounted to the proximal mount **2003.** The hemostatic seal **2001** can further comprise a distal mount **2009** comprising a distal aperture **2011** aligned with the central axis **805,** wherein the second end portion **853b** of the hemostatic spring **851** is mounted to the distal mount **2009.**

The hemostatic seal **2001** is configured to reduce the distance between the proximal mount **2003** and the distal mount **2009** along the central axis **805** to constrict the radial array of spring elements **801** to reduce a cross-sectional area of the central passage **1307.** As shown in **FIG. 20****,** in one embodiment, a drive nut **2050** can comprise internal threads **2051** threadedly engaging external threads **2006** of drive shaft **2004** of the proximal mount **2003.** Rotation of the drive nut **2050** about the central axis **805** can bias the proximal mount **2003** to reduce the distance between the proximal mount **2003** and the distal mount **2009** along the central axis **805** to constrict the radial array of spring segments **801.**

As shown, in each of the hemostatic seals **1301, 1801, 2001,** the proximal mount **1303, 1803, 2003** and distal mount **1309, 1809, 2009** may be provided with a circular ring reception aperture configured to snugly receive the tubular ends of the first and second end portions **853a, 853b** of the tubular members forming the hemostatic spring **851.** In some embodiments, the circular ring reception aperture may be deep enough to receive the entire end portions such that, once the end portions are fully inserted into the ring reception apertures, the distance between the proximal and distal mounts comprises the length of the spring segments. The circular ring reception apertures can be formed by boring, electrical discharge machining (EDM) or other techniques. Furthermore, the first and second end portions **853a, 853b** may be welded, press-fit, adhered or otherwise attached to the circular ring reception apertures. In some embodiments, the first and second end portions **853a, 853b** may be snuggly received within the circular ring reception apertures without being fixedly attached. Such embodiments may be desired in applications where the hemostatic spring will always be in compression such that attachment is not necessary. Avoiding attachment may be beneficial to permit replacing a damaged hemostatic spring or replacing a hemostatic spring with another hemostatic spring with a different spring constant that is more appropriate for the surgical device that will be used during a particular surgical procedure.

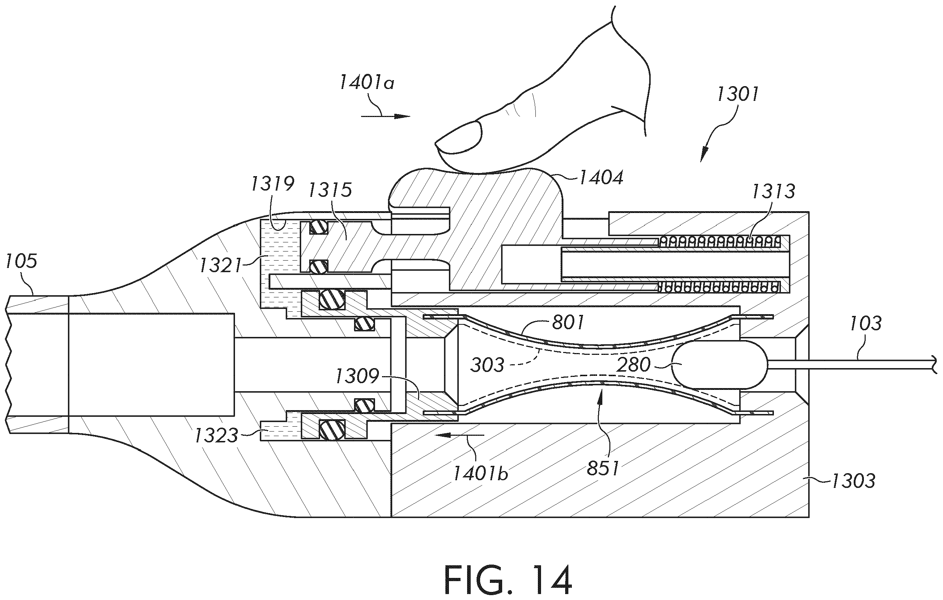

Methods of introducing a device **280** at the end of a catheter **103** into a vasculature of a patient with a hemostatic seal **101** will now be discussed with initial reference to **FIGS. 1-2** **and** **13** with the understanding that similar or identical methods may be provided in the other embodiments of the disclosure. The method can begin by percutaneously inserting the introducer sheath **105** into the vasculature of the patient wherein the introducer sheath **105** is in communication with the central passage **1307** of the hemostatic spring **851** and the hemostatic spring **851** is in axial compression to minimize leakage of fluid from the introducer sheath **105.** As shown in **FIG. 14****,** the method further comprises axially inserting the device **280** into the hemostatic seal **1301.** As further shown in **FIG. 14****,** a clinician can begin reducing a frictional force of inserting the device into the hemostatic seal **1301** by reducing the axial compression to dilate the hemostatic spring. For example, the clinician may begin applying a force in direction **1401a** to the trigger **1404** against the bias of compression spring **1313.** As a result, the trigger **1404** shifts in the direction **1401a** to compress the compression spring **1313** wherein the hydraulic fluid **1321** begins leaving the compression chamber **1323** and enters the hydraulic cylinder **1319.** The compression chamber **1323** therefore begins to decompress wherein the distal mount **1309** begins moving in direction **1401b.** Consequently, as the distal mount **1309** is moving apart from the proximal mount **1303,** the hemostatic spring **851** slightly dilates. Thus, the clinician can reduce the friction of inserting the device **280** into the hemostatic seal **1301** while still maintaining a proper sealed interface between the hemostatic seal **1301** and the device **280.**

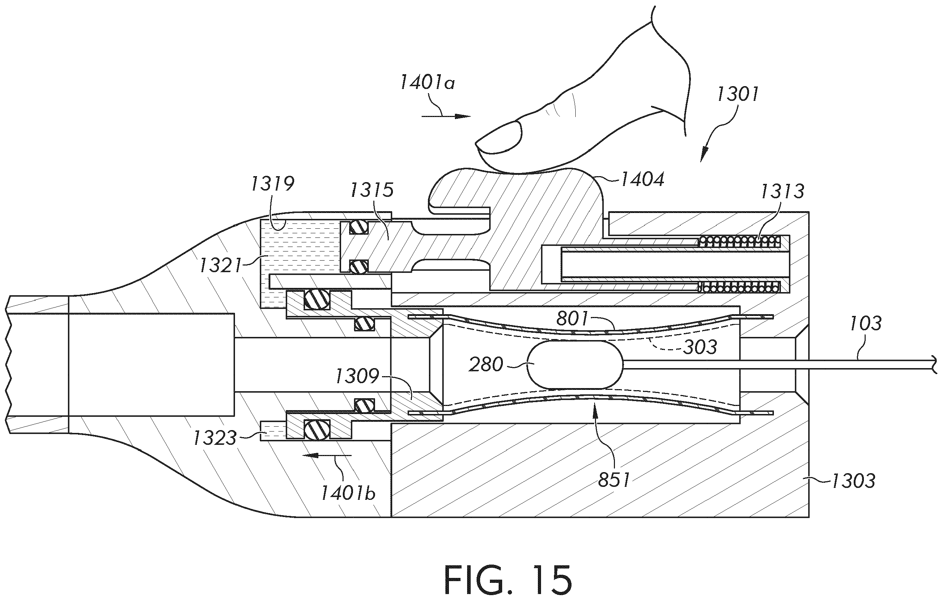

If further assistance is needed during insertion, as shown in **FIG. 15****,** the clinician may further dynamically reduce the frictional force of inserting the device **280** into the hemostatic seal **1301** by reducing the axial compression to further dilate the hemostatic spring **851.** For example, the clinician may apply additional force in direction **1401a** to the trigger **1404** against the bias of compression spring **1313.** In response to the additional application of force, the trigger **1404** again shifts in the direction **1401a** to further compress the compression spring **1313** and further draw the hydraulic fluid **1321** into the compression chamber hydraulic cylinder **1319** from the compression chamber **1323.** In response, the distal mount **1309** further shifts in the direction **1401b** to further move the distal mount **1309** farther apart from the proximal mount **1303.** Consequently, the hemostatic seal **1301** further dilates to further ease the frictional forces of inserting the device **280** while still providing sufficient constriction to seal against the surfaces of the device **280** to reduce fluid leakage from the introducer sheath **105** through the hemostatic seal **1301.**

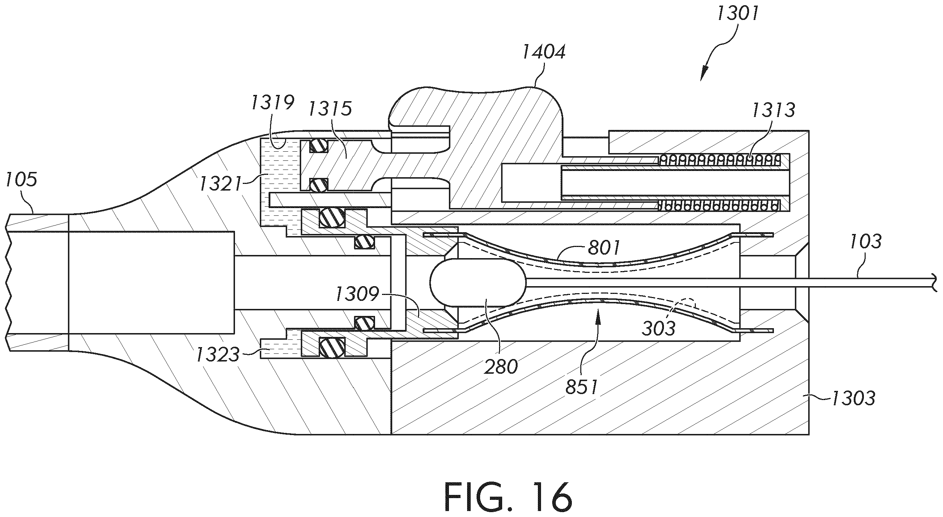

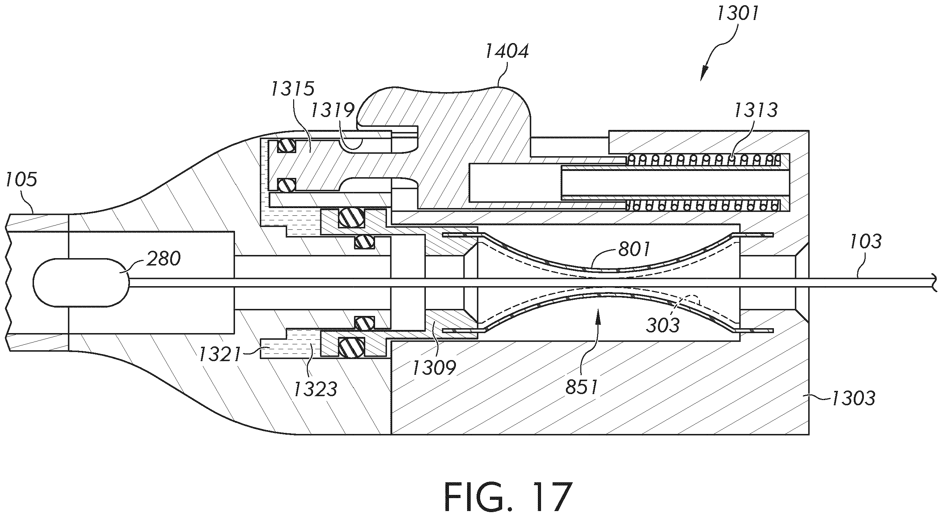

After reducing the frictional force, the method can subsequently include increasing the axial compression to constrict the hemostatic spring **851.** For example, **FIG. 15** represents the maximum device passage dimension that the hemostatic spring **851** achieves when the device **280** passes through the hemostatic seal **1301.** Once the device passes, as illustrated in **FIG. 16****,** the clinician may optionally release the trigger **1404.** Once released, the compression spring **1313** will automatically apply the appropriate pressure to the hydraulic fluid **1321** to maintain the proper seal against the device **280** with the hemostatic spring **851.** Indeed, as shown in **FIG. 16****,** a clinician can insert the device **280** past the maximum device passage dimension and thereafter release the trigger **1404.** The compression spring **1313** can then generate hydraulic pressure to maintain the axial compression of the hemostatic spring **251.** Indeed, the compression spring **1313** compresses the hydraulic fluid **1321** within the hydraulic cylinder **1319** with the linear piston **1315.** The hydraulic fluid **1321** then presses against the distal mount **1309** by entering the compression chamber **1323.** The pressurized hydraulic fluid **1321** therefore biases the distal mount **1309** toward the proximal mount **1303** to bias the hemostatic spring **251** to constrict to maintain the axial compression in the hemostatic spring **251.** As shown, the hemostatic spring **251** has constricted to seal against the device **280.** As shown in **FIG. 17****,** further biasing causes the hemostatic seal **1301** to seal against the exterior surfaces of the catheter **103** to further assist in reducing or preventing leaking of fluid from the introducer sheath **105.** As further shown in **FIG. 17****,** the method can comprise passing the device **280** into the introducer sheath **105** from the hemostatic seal **1301** and can then pass the device **280** from the introducer sheath **105** to the vasculature of a patient.

Methods of introducing a device **280** has been described with respect to the hemostatic seal **1301** with the understanding with similar or identical procedural steps can be performed with the other hemostatic seals **101, 1801, 2001** of the disclosure.

In some embodiments, methods of introducing the device **280** into the vasculature of a patient can comprise percutaneously inserting the introducer sheath **105** into the vasculature of the patient wherein the introducer sheath is provided with the hemostatic seal **101, 1301, 1801, 2001** comprising a hemostatic spring **251, 851, 1151, 1251** under axial compression to minimize leakage of fluid from the introducer sheath **105.** The method can further comprise axially inserting the device **208** into the hemostatic seal **101, 1301, 1801, 2001.** The method can further comprise reducing a frictional force of inserting the device **280** into the hemostatic seal **101, 1301, 1801, 2001** by reducing the axial compression to dilate the hemostatic spring **251, 851, 1151, 1251.**

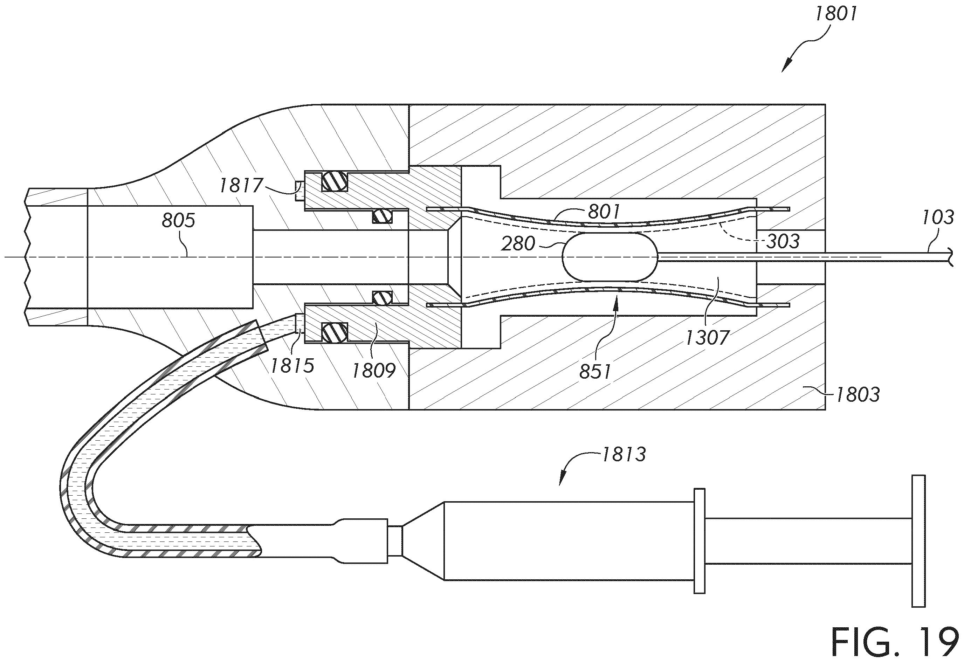

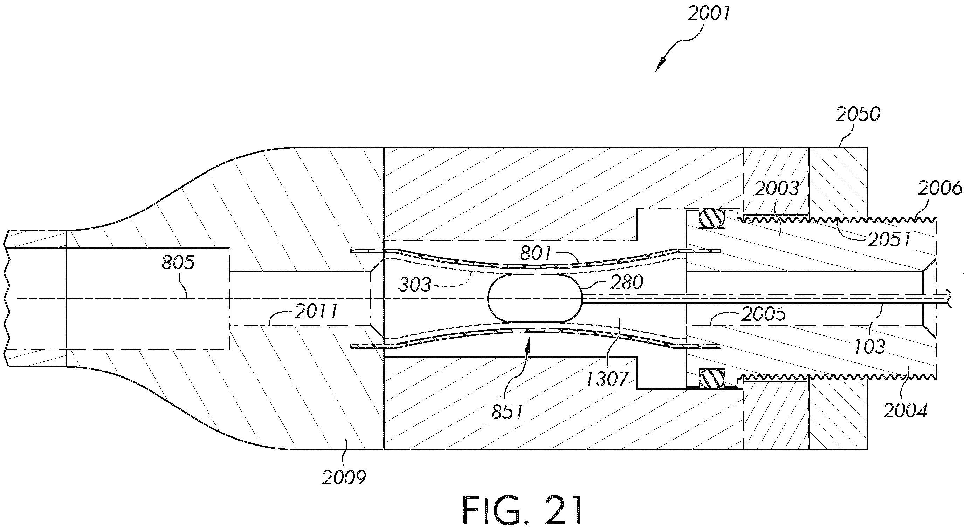

With initial schematical reference to **FIG. 3****,** reducing the frictional force of inserting the device **280** into the hemostatic seal **101** can be conducted by reducing the axial compression to dilate the hemostatic spring **251** by increasing the length of the hemostatic spring **251** from **L1** (see **FIG 2**) to **L2** (see **FIG. 3**). With reference to **FIG. 14****,** reducing the frictional force of inserting the device **280** into the hemostatic seal **1301** can be conducted by reducing the axial compression to dilate the hemostatic spring **851** by moving the trigger **1404** in direction **1401a** to increasing the length of the hemostatic spring **851** while compressing the compression spring **1313.** With reference to **FIG. 19****,** reducing the frictional force of inserting the device **280** into the hemostatic seal **1801** can be conducted by reducing the axial compression to dilate the hemostatic spring **851** by drawing hydraulic fluid into the syringe **1813** to increasing the length of the hemostatic spring **851.** With reference to **FIG. 21****,** reducing the frictional force of inserting the device **280** into the hemostatic seal **2001** can be conducted by reducing the axial compression to dilate the hemostatic spring **851** by rotating the drive nut **2050** to increase the length of the hemostatic spring **851.** Reducing the frictional force can begin at least prior to the hemostatic spring **251, 851, 1151, 1251** dilating to a maximum device passage dimension (see **FIGS. 3****,** **15****,** **19****, and** **21****)** that the hemostatic spring **251, 851, 1151, 1251** achieves when the device **280** passes through the hemostatic seal **101, 1301, 1801, 2001**

After reducing the frictional force, the method can comprise increasing the axial compression of the hemostatic spring to constrict the hemostatic spring. For example, in some embodiments, increasing the axial compression can occur after dilating the hemostatic spring to a maximum device passage dimension that the hemostatic spring achieves when the device passes through the hemostatic seal. For example, as shown in **FIG. 16****,** the trigger **1404** can be released wherein force can be applied from the compression spring **1313** to maintain the axial compression of the hemostatic spring **851.** Indeed, the compression spring **1313** can transfer force to compress the hydraulic fluid **1321.** The compressed hydraulic fluid **1321** can thereafter force the distal mount **1309** closer to the proximal mount **1303** to maintain axial compression on the hemostatic spring **851.** Referring to **FIG. 18****,** in further embodiments, the syringe **1813** may force pressurized hydraulic fluid to apply pressure to the distal mount **1809** to maintain axial compression on the hemostatic spring. Hydraulic fluid throughout the disclosure can comprise a wide range of incompressible fluids such as water, saline, oil, or other fluids. Referring to **FIG. 20****,** in further embodiments, the drive nut **2050** may be rotated to reduce the distance between the proximal mount **2003** and the distal mount **2009** to maintain axial compression on the hemostatic spring **851.**

The methods of the disclosure can conclude by passing the device **280** into the introducer sheath **105** from the hemostatic seal **101, 1301, 1801, 2001** and then passing the device **280** from the introducer sheath **105** to the vasculature of the patient.

In accordance with the disclosure, non-limiting aspects of the disclosure will now be described. Various combinations of the aspects can be provided in accordance with the disclosure.

Aspect 1. A hemostatic spring comprising a first end portion, a second end portion opposite the first end portion, and a radial array of spring segments positioned on a circular path about a central axis. Each spring segment of the radial array of spring segments comprises a first end, a second end, and a central location positioned between the first end and the second end. The first end of each spring segment is attached to the first end portion and each spring segment tapers toward the central location in a first direction of the central axis extending from the first end portion toward the second end portion. A second end of each spring segment is attached to the second end portion and each spring segment tapers toward the central location in a second direction of the central axis opposite the first direction. The central location of each spring segment is spaced on the circular path from the central location of each adjacent spring segment of the radial array of spring segments when a force is not applied to the radial array of spring segments.

Aspect 2. The hemostatic spring of Aspect 1, wherein each spring segment continuously tapers in the first direction from the first end of the corresponding spring segment toward the central location of the corresponding spring segment. Each spring segment further continuously tapers in the second direction from the second end of the corresponding spring segment toward the central location of the corresponding spring segment.

Aspect 3. The hemostatic spring of any one of Aspects 1-2, wherein the central location of each spring segment comprises a midpoint of the corresponding spring segment.

Aspect 4. The hemostatic spring of any one of Aspects 1-2, wherein the central location of each spring segment comprises a central segment of the corresponding spring segment.

Aspect 5. The hemostatic spring of Aspect 4, wherein the central segment comprises a width that is substantially constant along the central axis.

Aspect 6. The hemostatic spring of Aspect 4, wherein each spring segment continuously tapers from the first end to a first central waist of the central location. Each spring segment further continuously tapers from the second end to a second central waist of the central location. The central segment is positioned between the first central waist and the second central waist.

Aspect 7. The hemostatic spring of Aspect 6, wherein a width of the central segment is greater than a width of the first central waist and a width of the second central waist.

Aspect 8. The hemostatic spring of Aspect 7, wherein the width of the central segment is substantially constant along the central axis.

Aspect 9. The hemostatic spring of Aspect 1, wherein the first end of each spring segment is attached at a first end waist to the first end portion and the second end of each spring segment is attached at a second end waist to the second end portion. Each spring segment tapers in the first direction from a first intermediate portion toward the central location of the corresponding spring segment, and each spring segment tapers in the second direction of the central axis from a second intermediate portion toward the central location of the corresponding spring segment. The first end waist is positioned between the first end portion and the first intermediate portion, and the second end waist is positioned between the second end portion and the second intermediate portion. The first intermediate portion comprises a width greater than a width of the first end waist, and the second intermediate portion comprises a width greater than a width of the second end waist.

Aspect 10. The hemostatic spring of Aspect 9, wherein a width of the first end waist and the width of the second end waist are each greater than a width of the central location.

Aspect 11. The hemostatic spring of Aspect 9, wherein the central location of each spring segment comprises a central segment of the corresponding spring segment.

Aspect 12. The hemostatic spring of Aspect 11, wherein the central segment comprises a width that is substantially constant along the central axis.

Aspect 13. The hemostatic spring of Aspect 12, wherein a width of the first end waist and the width of the second end waist are each greater than the width of the central segment.

Aspect 14. The hemostatic spring of any one of Aspects 9-13, wherein each spring segment continuously tapers in the first direction from the first intermediate portion to the central location of the corresponding spring segment. Each spring segment further continuously tapers in the second direction from the second end intermediate portion to the central location of the corresponding spring segment.

Aspect 15. The hemostatic spring of any one of Aspects 1-14, wherein each spring segment of the radial array of spring segments is substantially straight and extends parallel to the central axis when a force is not applied to the radial array of spring segments.

Aspect 16. The hemostatic spring of any one of Aspects 1-15, wherein the first end portion and the second end portion each comprise a continuous circular cylindrical tube.

Aspect 17. The hemostatic spring of any one of Aspects 1-16, wherein a thickness of the first end portion, a thickness of the second end portion, and a thickness of each spring segment are approximately equal.

Aspect 18. The hemostatic spring of any one of Aspects 1-17, further comprising a sleeve positioned within an interior of the hemostatic spring.

Aspect 19. The hemostatic spring of Aspect 18, wherein the sleeve comprises an elastomeric layer.

Aspect 20. The hemostatic spring of any one of Aspects 18-19, wherein the sleeve comprises a polymer layer.

Aspect 21. A hemostatic seal comprising a hemostatic spring comprising a first end portion, a second end portion opposite the first end portion, and a radial array of spring segments positioned on a circular path about a central axis. Each spring segment of the radial array of spring segments comprises a first end, a second end, and a central location positioned between the first end and the second end. The first end of each spring segment is attached to the first end portion and each spring segment tapers toward the central location in a first direction of the central axis extending from the first end portion toward the second end portion. A second end of each spring segment is attached to the second end portion and each spring segment tapers toward the central location in a second direction of the central axis opposite the first direction. The central location of each spring segment is spaced on the circular path from the central location of each adjacent spring segment of the radial array of spring segments when a force is not applied to the radial array of spring segments. The hemostatic seal further comprises a proximal mount comprising a proximal aperture aligned with the central axis extending through a central passage of the hemostatic spring, wherein the first end portion of the hemostatic spring is mounted to the proximal mount. The hemostatic seal further comprises a distal mount comprising a distal aperture aligned with the central axis, wherein the second end portion of the hemostatic spring is mounted to the distal mount. The hemostatic seal is configured to reduce the distance between the proximal mount and the distal mount along the central axis to constrict the radial array of spring elements to reduce a cross-sectional area of the central passage.

Aspect 22. The hemostatic seal of Aspect 21, further comprising a spring biasing at least one of the proximal mount and the distal mount to reduce the distance between the proximal mount and the distal mount along the central axis to constrict the radial array of spring elements.

Aspect 23. The hemostatic seal of Aspect 22, further comprising an actuator configured to act against the bias of the spring to dilate the radial array of spring segments to increase a cross-sectional area of the central passage.

Aspect 24. The hemostatic seal of Aspect 23, wherein the actuator comprises a linear trigger.

Aspect 25. The hemostatic seal of any one of Aspects 23-24, wherein the actuator comprises a linear piston.

Aspect 26. The hemostatic seal of Aspect 21, further comprising a fluid that is hydraulically compressed to bias at least one of the proximal mount and the distal mount to reduce the distance between the proximal mount and the distal mount along the central axis to constrict the radial array of spring elements.

Aspect 27. The hemostatic seal of any one of Aspects 21-26, wherein each spring segment continuously tapers in the first direction from the first end of the corresponding spring segment toward the central location of the corresponding spring segment. Each spring segment further continuously tapers in the second direction from the second end of the corresponding spring segment toward the central location of the corresponding spring segment.

Aspect 28. The hemostatic seal of any one of Aspects 21-27, wherein the central location of each spring segment comprises a midpoint of the corresponding spring segment.

Aspect 29. The hemostatic seal of any one of Aspects 21-27, wherein the central location of each spring segment comprises a central segment of the corresponding spring segment.

Aspect 30. The hemostatic seal of Aspect 29, wherein the central segment comprises a width that is substantially constant along the central axis.

Aspect 31. The hemostatic seal of Aspect 29, wherein each spring segment continuously tapers from the first end to a first central waist of the central location. Each spring segment further continuously tapers from the second end to a second central waist of the central location. The central segment is positioned between the first central waist and the second central waist.

Aspect 32. The hemostatic seal of Aspect 31, wherein the width of the central segment is greater than a width of the first central waist and a width of the second central waist.

Aspect 33. The hemostatic seal of Aspect 32, wherein the width of the central segment is substantially constant along the central axis.

Aspect 34. The hemostatic seal of any one of Aspects 21-26, wherein the first end of each spring segment is attached at a first end waist to the first end portion and the second end of each spring segment is attached at a second end waist to the second end portion. Each spring segment tapers in the first direction from a first intermediate portion toward the central location of the corresponding spring segment. Each spring segment further tapers in the second direction of the central axis from a second intermediate portion toward the central location of the corresponding spring segment. The first end waist is positioned between the first end portion and the first intermediate portion. The second end waist is positioned between the second end portion and the second intermediate portion. The first intermediate portion comprises a width greater than a width of the first end waist, and the second intermediate portion comprises a width greater than a width of the second end waist.

Aspect 35. The hemostatic seal of Aspect 34, wherein a width of the first end waist and the width of the second end waist are each greater than a width of the central location.

Aspect 36. The hemostatic seal of Aspect 34, wherein the central location of each spring segment comprises a central segment of the corresponding spring segment.

Aspect 37. The hemostatic seal of Aspect 36, wherein the central segment comprises a width that is substantially constant along the central axis.

Aspect 38. The hemostatic seal of Aspect 37, wherein a width of the first end waist and the width of the second end waist are each greater than the width of the central segment.

Aspect 39. The hemostatic seal of any one of Aspects 34-38, wherein each spring segment continuously tapers in the first direction from the first intermediate portion to the central location of the corresponding spring segment, and each spring continuously tapers in the second direction from the second end intermediate portion to the central location of the corresponding spring segment.

Aspect 40. The hemostatic seal of any one of Aspects 21-39, wherein each spring segment of the radial array of spring segments is substantially straight and extends parallel to the central axis when a force is not applied to the radial array of spring segments.

Aspect 41. The hemostatic seal of any one of Aspects 21-40, wherein the first end portion and the second end portion each comprise a continuous circular cylindrical tube.

Aspect 42. The hemostatic seal of any one of Aspects 21-41, wherein a thickness of the first end portion, a thickness of the second end portion, and a thickness of each spring segment are approximately equal.

Aspect 43. The hemostatic seal of any one of Aspects 21-42, further comprising a sleeve positioned within an interior of the hemostatic spring.

Aspect 44. The hemostatic seal of Aspect 43, wherein the sleeve comprises an elastomeric layer.

Aspect 45. The hemostatic seal of any one of Aspects 43-44, wherein the sleeve comprises a polymer layer.

Aspect 46. A method of introducing a device into a vasculature of a patient with the hemostatic seal of Aspect 21, comprising percutaneously inserting an introducer sheath into the vasculature of the patient, wherein the introducer sheath is in communication with the central passage, and the hemostatic spring is under an axial compression to minimize leakage of fluid from the introducer sheath. The method further comprises axially inserting the device into the hemostatic seal and reducing a frictional force of inserting the device into the hemostatic seal by reducing the axial compression to dilate the hemostatic spring. The method further comprises passing the device into the introducer sheath from the hemostatic seal, and passing the device from the introducer sheath to the vasculature of the patient.

Aspect 47. The method of Aspect 46, wherein, after reducing the frictional force, increasing the axial compression to constrict the hemostatic spring.

Aspect 48. The method of Aspect 47, wherein increasing the axial compression occurs after dilating the hemostatic spring to a maximum device passage dimension that the hemostatic spring achieves when the device passes through the hemostatic seal.

Aspect 49. The method of Aspect 46, wherein reducing the frictional force begins at least prior to the hemostatic spring dilating to a maximum device passage dimension that the hemostatic spring achieves when the device passes through the hemostatic seal.

Aspect 50. The method of Aspect 49, wherein after reducing the frictional force, reapplying the axial compression to constrict the hemostatic spring.

Aspect 51. The method of Aspect 50, wherein reapplying the axial compression occurs after dilating the hemostatic spring to the maximum device passage dimension.

Aspect 52. The method of any one of Aspects 46-51, further comprising applying force from a compression spring to maintain the axial compression of the hemostatic spring.

Aspect 53. The method of Aspect 52, wherein reducing the frictional force of inserting the device into the hemostatic seal comprises applying a force to compress the compression spring.

Aspect 54. The method of any one of Aspects 52-53, wherein the compression spring generates hydraulic pressure to maintain the axial compression of the hemostatic spring.

Aspect 55. The method of any one of Aspects 46-51, further comprising applying hydraulic pressure to maintain the axial compression in the hemostatic spring.

Aspect 56. The method of Aspect 55, wherein the hydraulic pressure is maintained by a syringe.

Aspect 57. The method of anyone of Aspects 46-56, wherein the hemostatic spring is under the axial compression to minimize the leakage of fluid from the introducer sheath by biasing at least one of the proximal mount and the distal mount to reduce a distance between the proximal mount and the distal mount along the central axis to constrict the radial array of spring elements.

Aspect 58. The method of Aspect 57, further comprising acting against the bias of the at least one of the proximal mount and the distal mount to dilate the radial array of spring segments to increase a cross-sectional area of the central passage.

Aspect 59. A method of introducing a device into a vasculature of a patient comprising percutaneously inserting an introducer sheath into the vasculature of the patient wherein the introducer sheath is provided with a hemostatic seal comprising a hemostatic spring under an axial compression to minimize leakage of fluid from the introducer sheath. The method further comprises axially inserting the device into the hemostatic seal, and reducing a frictional force of inserting the device into the hemostatic seal by reducing the axial compression to dilate the hemostatic spring. The method further comprises passing the device into the introducer sheath from the hemostatic seal and passing the device from the introducer sheath to the vasculature of the patient.

Aspect 60. The method of Aspect 59, wherein, after reducing the frictional force, increasing the axial compression to constrict the hemostatic spring.

Aspect 61. The method of Aspect 60, wherein increasing the axial compression occurs after dilating the hemostatic spring to a maximum device passage dimension that the hemostatic spring achieves when the device passes through the hemostatic seal.

Aspect 62. The method of Aspect 59, wherein reducing the frictional force begins at least prior to the hemostatic spring dilating to a maximum device passage dimension that the hemostatic spring achieves when the device passes through the hemostatic seal.

Aspect 63. The method of Aspect 62, wherein after reducing the frictional force, reapplying the axial compression to constrict the hemostatic spring.

Aspect 64. The method of Aspect 63, wherein reapplying the axial compression occurs after dilating the hemostatic spring to the maximum device passage dimension.

Aspect 65. The method of any one of Aspects 59-64, further comprising applying force from a compression spring to maintain the axial compression of the hemostatic spring.

Aspect 66. The method of Aspect 65, wherein reducing the frictional force of inserting the device into the hemostatic seal comprises applying a force to compress the compression spring.

Aspect 67. The method of any one of Aspects 65-66, wherein the compression spring generates hydraulic pressure to maintain the axial compression of the hemostatic spring.

Aspect 68. The method of any one of Aspects 59-64, further comprising applying hydraulic pressure to maintain the axial compression in the hemostatic spring.

Aspect 69. The method of Aspect 68, wherein the hydraulic pressure is maintained by a syringe.

Further disclosed herein is the subject matter of the following clauses:
1. A hemostatic spring comprising:
   a first end portion;
   a second end portion opposite the first end portion;
   a radial array of spring segments positioned on a circular path about a central axis, each spring segment of the radial array of spring segments comprising a first end, a second end, and a central location positioned between the first end and the second end, wherein the first end of each spring segment is attached to the first end portion and each spring segment tapers toward the central location in a first direction of the central axis extending from the first end portion toward the second end portion, and a second end of each spring segment is attached to the second end portion and each spring segment tapers toward the central location in a second direction of the central axis opposite the first direction,
   wherein the central location of each spring segment is spaced on the circular path from the central location of each adjacent spring segment of the radial array of spring segments when a force is not applied to the radial array of spring segments.
2. The hemostatic spring of clause 1, wherein each spring segment continuously tapers in the first direction from the first end of the corresponding spring segment toward the central location of the corresponding spring segment, and each spring segment continuously tapers in the second direction from the second end of the corresponding spring segment toward the central location of the corresponding spring segment.
3. The hemostatic spring of clause 1 or any preceding clause, wherein the central location of each spring segment comprises a midpoint of the corresponding spring segment.
4. The hemostatic spring of clause 1 or any preceding clause, wherein the central location of each spring segment comprises a central segment of the corresponding spring segment.
5. The hemostatic spring of clause 4, wherein the central segment comprises a width that is substantially constant along the central axis.
6. The hemostatic spring of clause 4 or 5, wherein each spring segment continuously tapers from the first end to a first central waist of the central location, and continuously tapers from the second end to a second central waist of the central location, wherein the central segment is positioned between the first central waist and the second central waist.
7. The hemostatic spring of clause 6, wherein a width of the central segment is greater than a width of the first central waist and a width of the second central waist.
8. The hemostatic spring of clause 7, wherein the width of the central segment is substantially constant along the central axis.
9. The hemostatic spring of clause 1 or any preceding clause, wherein the first end of each spring segment is attached at a first end waist to the first end portion and the second end of each spring segment is attached at a second end waist to the second end portion, wherein the each spring segment tapers in the first direction from a first intermediate portion toward the central location of the corresponding spring segment, and each spring segment tapers in the second direction of the central axis from a second intermediate portion toward the central location of the corresponding spring segment, wherein the first end waist is positioned between the first end portion and the first intermediate portion, the second end waist is positioned between the second end portion and the second intermediate portion, the first intermediate portion comprises a width greater than a width of the first end waist, and the second intermediate portion comprises a width greater than a width of the second end waist.
10. The hemostatic spring of clause 9, wherein a width of the first end waist and the width of the second end waist are each greater than a width of the central location.
11. The hemostatic spring of clause 9 or 10, wherein the central location of each spring segment comprises a central segment of the corresponding spring segment.
12. The hemostatic spring of clause 11, wherein the central segment comprises a width that is substantially constant along the central axis.
13. The hemostatic spring of clause 12, wherein a width of the first end waist and the width of the second end waist are each greater than the width of the central segment.
14. The hemostatic spring of clause 9, wherein each spring segment continuously tapers in the first direction from the first intermediate portion to the central location of the corresponding spring segment, and each spring continuously tapers in the second direction from the second end intermediate portion to the central location of the corresponding spring segment.
15. The hemostatic spring of clause 1 or any preceding clause, wherein each spring segment of the radial array of spring segments is substantially straight and extends parallel to the central axis when a force is not applied to the radial array of spring segments.
16. The hemostatic spring of clause 1 or any preceding clause, wherein the first end portion and the second end portion each comprise a continuous circular cylindrical tube.
17. The hemostatic spring of clause 1 or any preceding clause, wherein a thickness of the first end portion, a thickness of the second end portion, and a thickness of each spring segment are approximately equal.
18. The hemostatic spring of clause 1 or any preceding clause, further comprising a sleeve positioned within an interior of the hemostatic spring.
19. The hemostatic spring of clause 18, wherein the sleeve comprises an elastomeric layer.
20. The hemostatic spring of clause 18, wherein the sleeve comprises a polymer layer.

Further disclosed herein are hemostatic springs and hemostatic seals. Hemostatic springs and hemostatic seals comprising hemostatic springs facilitate introducing a surgical device into the vasculature of a patient during a surgical procedure. Each hemostatic spring comprises a radial array of spring segments positioned on a circular path about a central axis. Each hemostatic seal is configured to reduce an axial length of the hemostatic spring to constrict the radial array of spring segments to reduce a cross-sectional area of the central passage. Methods include axially inserting the surgical device into the hemostatic seal while compression from the hemostatic seal minimizes fluid leakage. Methods can further include reducing a frictional force of inserting the device into the hemostatic seal by reducing the axial compression to dilate the hemostatic spring.

It should be understood that while various aspects have been described in detail relative to certain illustrative and specific examples thereof, the present disclosure should not be considered limited to such, as numerous modifications and combinations of the disclosed features are possible without departing from the scope of the following claims.

## Claims

1. A hemostatic spring (251, 851, 1151, 1251) comprising:
a first end portion (253a, 853a, 1153a, 1253a);
a second end portion (253b, 853b, 1153b, 1253b) opposite the first end portion (253a, 853a, 1153a, 1253a);
a radial array of spring segments (501a-h, 801, 1101, 1201) positioned on a circular path (503) about a central axis (505, 805, 1105, 1205), each spring segment (501a-h, 801, 1101, 1201) of the radial array of spring segments (501a-h, 801, 1101, 1201) comprising a first end (301a, 901a, 1101a, 1201a), a second end (301b, 901b, 1101b, 1201b), and a central location (301c, 901 c, 1101c, 1201c) positioned between the first end (301a, 901a, 1101a, 1201a) and the second end (301b, 901b, 1101b, 1201b), wherein the first end (301a, 901a, 1101a, 1201a) of each spring segment (501a-h, 801, 1101, 1201) is attached to the first end portion (253a, 853a, 1153a, 1253a) and each spring segment (501a-h, 801, 1101, 1201) tapers toward the central location (301c, 901c, 1101c, 1201c) in a first direction (401a, 805a, 1105a, 1205a) of the central axis (505, 805, 1105, 1205) extending from the first end portion (253a, 853a, 1153a, 1253a) toward the second end portion (253b, 853b, 1153b, 1253b), and a second end (301b, 901b, 1101b, 1201b) of each spring segment (501a-h, 801, 1101, 1201) is attached to the second end portion (253b, 853b, 1153b, 1253b) and each spring segment (501a-h, 801, 1101, 1201) tapers toward the central location (301c, 901c, 1101c, 1201c) in a second direction (401b, 805b, 1105b, 1205b) of the central axis (505, 805, 1105, 1205) opposite the first direction (401a, 805a, 1105a, 1205a),
wherein the central location (301c, 901c, 1101c, 1201c) of each spring segment (501a-h, 801, 1101, 1201) is spaced on the circular path (503) from the central location (301c, 901c, 1101c, 1201c) of each adjacent spring segment of the radial array of spring segments (501a-h, 801, 1101, 1201) when a force is not applied to the radial array of spring segments (501a-h, 801, 1101, 1201).

2. The hemostatic spring (251, 851, 1151, 1251) of claim 1, wherein each spring segment (501a-h, 801, 1101, 1201) continuously tapers in the first direction (401a, 805a, 1105a, 1205a) from the first end (301a, 901a, 1101a, 1201a) of the corresponding spring segment (501a-h, 801, 1101, 1201) toward the central location (301c, 901c, 1101c, 1201c) of the corresponding spring segment (501a-h, 801, 1101, 1201), and each spring segment (501a-h, 801, 1101, 1201) continuously tapers in the second direction (401b, 805b, 1105b, 1205b) from the second end (301b, 901b, 1101b, 1201b) of the corresponding spring segment (501a-h, 801, 1101, 1201) toward the central location (301c, 901c, 1101c, 1201c) of the corresponding spring segment (501ah, 801, 1101, 1201).

3. The hemostatic spring (1151, 1251) of any one of claims 1-2, wherein the central location (1101c, 1201c) of each spring segment (1101, 1201) comprises a central segment (1102, 1202) of the corresponding spring segment (1101, 1201).

4. The hemostatic spring (1151, 1251) of claim 3, wherein the central segment (1102, 1202) comprises a width (W) that is substantially constant along the central axis (1105, 1205).

5. The hemostatic spring (1151) of claim 3 or 4, wherein each spring segment (1101) continuously tapers from the first end (1101a) to a first central waist (1104a) of the central location (1101c), and continuously tapers from the second end (1101b) to a second central waist (1104b) of the central location (1101c), wherein the central segment (1102) is positioned between the first central waist (1104a) and the second central waist (1104b).

6. The hemostatic spring (1251) of any preceding claim, wherein the first end (1201a) of each spring segment (1201) is attached at a first end waist (1281a) to the first end portion (1253a) and the second end (1201b) of each spring segment (1201) is attached at a second end waist (1281b) to the second end portion (1253b), wherein the each spring segment (1201) tapers in the first direction (1205a) from a first intermediate portion (1283a) toward the central location (1201c) of the corresponding spring segment (1201), and each spring segment (1201) tapers in the second direction (1205b) of the central axis (1205) from a second intermediate portion (1283b) toward the central location (1201c) of the corresponding spring segment (1201), wherein the first end waist (1281a) is positioned between the first end portion (1253a) and the first intermediate portion (1283a), the second end waist (1281b) is positioned between the second end portion (1253b) and the second intermediate portion (1283b), the first intermediate portion (1283a) comprises a width (1284a) greater than a width (1282a) of the first end waist (1281a), and the second intermediate portion (1283b) comprises a width (1284a) greater than a width (1282a) of the second end waist (1281b).

7. A hemostatic seal (101, 1301, 1801, 2001) comprising:
a hemostatic spring (251, 851, 1151, 1251) comprising:
a first end portion (253a, 853a, 1153a, 1253a);
a second end portion (253b, 853b, 1153b, 1253b) opposite the first end portion (253a, 853a, 1153a, 1253a);
a radial array of spring segments (501a-h, 801, 1101, 1201) positioned on a circular path (503) about a central axis (505, 805, 1105, 1205), each spring segment (501a-h, 801, 1101, 1201) of the radial array of spring segments (501a-h, 801, 1101, 1201) comprising a first end (301a, 901a, 1101a, 1201a), a second end (301b, 901b, 1101b, 1201b), and a central location (301c, 901c, 1101c, 1201c) positioned between the first end (301a, 901a, 1101a, 1201a) and the second end (301b, 901b, 1101b, 1201b), wherein the first end (301a, 901a, 1101a, 1201a) of each spring segment (501a-h, 801, 1101, 1201) is attached to the first end portion (253a, 853a, 1153a, 1253a) and each spring segment (501a-h, 801, 1101, 1201) tapers toward the central location (301c, 901c, 1101c, 1201c) in a first direction (401a, 805a, 1105a, 1205a) of the central axis (505, 805, 1105, 1205) extending from the first end portion (253b, 853b, 1153b, 1253b) toward the second end portion (253b, 853b, 1153b, 1253b), and a second end (301b, 901b, 1101b, 1201b) of each spring segment (501a-h, 801, 1101, 1201) is attached to the second end portion (253b, 853b, 1153b, 1253b) and each spring segment (501a-h, 801, 1101, 1201) tapers toward the central location (301c, 901c, 1101c, 1201c) in a second direction (401b, 805b, 1105b, 1205b) of the central axis (505, 805, 1105, 1205) opposite the first direction (401a, 805a, 1105a, 1205a),
wherein the central location (301c, 901c, 1101c, 1201c) of each spring segment (501a-h, 801, 1101, 1201) is spaced on the circular path (503) from the central location (301c, 901c, 1101c, 1201c) of each adjacent spring segment (501a-h, 801, 1101, 1201) of the radial array of spring segments (501a-h, 801, 1101, 1201) when a force is not applied to the radial array of spring segments (501a-h, 801, 1101, 1201);
a proximal mount (1303, 1803, 2003) comprising a proximal aperture (1305, 1805, 2005) aligned with the central axis (505, 805, 1105, 1205) extending through a central passage (1307) of the hemostatic spring (251, 851, 1151, 1251), wherein the first end portion (253a, 853a, 1153a, 1253a) of the hemostatic spring (251, 851, 1151, 1251) is mounted to the proximal mount (1303, 1803, 2003);
a distal mount (1309, 1809, 2009) comprising a distal aperture (1311, 1811, 2011) aligned with the central axis (505, 805, 1105, 1205), wherein the second end portion (253b, 853b, 1153b, 1253b) of the hemostatic spring (251, 851, 1151, 1251) is mounted to the distal mount (1309, 1809, 2009),
wherein the hemostatic seal (101, 1301, 1801, 2001) is configured to reduce the distance between the proximal mount (1303, 1803, 2003) and the distal mount (1309, 1809, 2009) along the central axis (505, 805, 1105, 1205) to constrict the radial array of spring elements (501a-h, 801, 1101, 1201) to reduce a cross-sectional area of the central passage (1307).

8. The hemostatic seal (1301) of claim 7, further comprising a spring (1313) biasing at least one of the proximal mount (1303) and the distal mount (1309) to reduce the distance between the proximal mount (1303) and the distal mount (1309) along the central axis (505, 805, 1105, 1205) to constrict the radial array of spring elements (501a-h, 801, 1101, 1201).

9. The hemostatic seal (1301) of claim 8, further comprising an actuator (1317) configured to act against the bias of the spring (1313) to dilate the radial array of spring segments (501a-h, 801, 1101, 1201) to increase a cross-sectional area of the central passage (1307).

10. The hemostatic seal (1301, 1801) of any one of claims 7 to 9, further comprising a fluid (1321, 1815) that is hydraulically compressed to bias at least one of the proximal mount (1303, 1803) and the distal mount (1309 1809) to reduce the distance between the proximal mount (1303, 1803) and the distal mount (1309, 1809) along the central axis (505, 805, 1105, 1205) to constrict the radial array of spring elements (501a-h, 801, 1101, 1201).

11. A method of introducing a device (208) into a vasculature of a patient comprising:
percutaneously inserting an introducer sheath (105) into the vasculature of the patient wherein the introducer sheath (105) is provided with a hemostatic seal (101, 1301, 1801, 2001) comprising a hemostatic spring (251, 851, 1151, 1251) under an axial compression to minimize leakage of fluid from the introducer sheath (105);
axially inserting the device (208) into the hemostatic seal (101, 1301, 1801, 2001);
reducing a frictional force of inserting the device (208) into the hemostatic seal (101, 1301, 1801, 2001) by reducing the axial compression to dilate the hemostatic spring (251, 851, 1151, 1251);
passing the device (208) into the introducer sheath (105) from the hemostatic seal (101, 1301, 1801, 2001); and
passing the device (208) from the introducer sheath (105) to the vasculature of the patient.

12. The method of claim 11, further comprising applying force from a compression spring (1313) to maintain the axial compression of the hemostatic spring (1151).

13. The method of claim 12, wherein reducing the frictional force of inserting the device (208) into the hemostatic seal (101, 1301, 1801, 2001) comprises applying a force to compress the compression spring (1313).

14. The method of any one of claims 12-13, wherein the compression spring (1313) generates hydraulic pressure to maintain the axial compression of the hemostatic spring (251, 851, 1151, 1251).

15. The method of claim 11, further comprising applying hydraulic pressure to maintain the axial compression in the hemostatic spring (251, 851, 1151, 1251).
